# EUROPÄISCHE PATENTANMELDUNG

(11) **EP 1 880 725 A1**
(43) Veröffentlichungstag der Anmeldung: **23.01.2008**
(21) Anmeldenummer: 07012448.2
(22) Anmeldetag: 21.06.2007
(51) Int. Cl.: A61K 31/5513, A61K 31/554, A61K 9/08, A61K 47/10, A61P 25/18

(54) **Flüssige Formulierung enthaltend Clozapin, Quetiapin, einen Salz von Quetiapin und/oder Olanzapin zur oralen Verabreichung**

(30) Priorität: 13.07.2006 DE 102006032426
(71) Anmelder: Neuraxpharm Arzneimittel GmbH u. Co. KG, 40764 Langenfeld (DE)
(72) Erfinder: Sewarte-Ross, Günter, Dr., 44145 Dortmund (DE)
(74) Vertreter: Beszédes, Stephan G.

(57) **Zusammenfassung**

Die Erfindung betrifft die Verwendung von Zusammensetzungen mit
a) 20 bis 100 mg/ml Clozapin, 25 bis 100 mg/ml Quetiapin, 25 bis 100 mg/ml (ausgedrückt als Quetiapinbase) von 1 oder mehr schwer oder sehr schwer löslichen Säureadditionssalz(en) von Quetiapin und/oder 1 bis 10 mg/ml Olanzapin als Wirkstoff(en),
b) 0 bis 20 mg/ml Benzylalkohol und/oder Phenylethylalkohol,
c) 25 bis 150 mg/ml Propylenglykol,
d) 0 bis 50 mg/ml von 1 oder mehr ätherischen Öl(en), Aroma bzw. Aromen und/oder Süßungsmittel(n) und
e) Rest bis je 1 ml von 1 oder mehr flüssigen Polyethylenglykol(en),

wobei an Stelle von c) oder zusätzlich zu c) bis zu 50 mg/ml Ethanol und/oder bis zu 0,5 Gew.-%, bezogen auf den/die Wirkstoff (e), von 1 oder mehr C₃₋₄-Alkanol (en) vorliegen können, zur Herstellung von Lösungen zur oralen Verabreichung.

Durch die Erfindung werden gut tropfbare stabile Lösungen erhalten.

## Beschreibung

Die Erfindung betrifft die Verwendung von Zusammensetzungen von Clozapin, Quetiapin, schwer oder sehr schwer löslichen Säureadditionssalzen von Quetiapin und/oder Olanzapin zur Herstellung von Lösungen zur oralen Verabreichung.

Atypische Neuroleptika erlangen zunehmend Bedeutung.

Solche, wie Clozapin, Quetiapin einschließlich seiner Säureadditionssalze und Olanzapin, werden zur Behandlung therapieresistenter Schizophrenie und schizophrener Patienten, die mit schweren, nicht zu behandelnden neurologischen unerwünschten Reaktionen auf andere Neuroleptika einschließlich atypischer Neuroleptika reagieren, sowie bei Psychosen im Verlauf eines Morbus Parkinson nach Versagen der Standardtherapie angewandt.

Lösungen bieten den Vorteil einer erleichterten Einnahme für Patienten, die feste Darreichungsformen schlecht schlucken können. Teilweise, insbesondere bei Patienten mit Morbus Parkinson, werden Clozapin, Quetiapin und Olanzapin sehr niedrig dosiert. Grundsätzlich sollte die niedrigst wirksame Dosis verabreicht werden. Auch bei niedriger Tagesdosis ist eine Verteilung auf mehrere Einzeldosen sinnvoll, um Nebenwirkungen auch bei sensiblen Patienten so weit wie möglich zu vermeiden. Hier bietet sich die Tropflösung mit der Möglichkeit der tropfenweisen Dosierung zum Beispiel in 1 mg-Schritten feinere Möglichkeiten der Dosisfindung, als es beispielsweise bei Tabletten von Clozapin und Quetiapin mit Vielfachen von 12,5 mg bzw. 25 mg (1/2 oder 1 Tablette der niedrigsten Wirkstärke) der Fall ist.

Die Wirkstoffe Clozapin, Quetiapin und einige seiner Salze, wie das Fumarat und Maleat, und Olanzapin sind praktisch wasserunlöslich. Übliche Lösungsvermittler, wie Sorbit oder Glycerin, reichen nicht aus, um Clozapin, Quetiapin und seine schwer oder sehr schwer löslichen Salze und Olanzapin in wäßrigen Medien zu lösen. In stark sauren Medien (pH-Wert < 2) lösen sich zwar Clozapin, Quetiapin und Olanzapin, sie werden aber schnell hydrolysiert.

Die Standarddarreichungsformen, wie Tabletten, Filmtabletten und Kapseln, haben bei bestimmten Patientengruppen Nachteile. Zum einen gibt es immer mehr Patienten, die Tabletten oder Kapseln schlecht oder gar nicht schlucken können, zum anderen ist häufig für besonders sensible Patienten (Kinder und Jugendliche, ältere Patienten, Patienten mit bestimmten Begleiterkrankungen oder notwendigen Co-Medikationen) eine feinere Dosisabstimmung wünschenswert, als es mit diesen Darreichungsformen möglich ist. Bisher bekannte Lösungsansätze, wie eine Suspension (WO 2005/007168 A1) oder Schmelztabletten (beispielsweise ZYPREXA VELOTAB^{®} mit dem Wirkstoff Olanzapin) lösen nur den ersten Teil der obigen Probleme. Das Zerreiben von Tabletten ist häufig keine Alternative, da der durchschnittliche Patient kaum geeignete Geräte zur Verfügung hat, hygienische Risiken und Stabilitätsprobleme könnten noch dazukommen. Auch gibt es Hinweise, daß die Resorption von zerriebenen und von als Ganzes verabreichten Clozapin-Tabletten sich signifikant unterscheidet.

Aus WO 2005/007168 A1 sind also typische Suspensionsformulierungen auch von Clozapin unter einer großen Zahl von Wirkstoffen bekannt. Zwar kann auch Propylenglykol enthalten sein, es handelt sich aber in allen Fällen um wäßrige Suspensionen.

In WO 96/18622 sind Lösungen von Wirkstoffen in Polyethylenglykol, Ölen, Ethanol oder Glycerin (Seite 9, vorletzter Absatz) angegeben. Unter eine allgemeine Formel mit vielen Substituenten und deren Kombinationen fällt auch Clozapin [Formel I mit X₁ = NH, X₂-- = N=, R₁ und R₃ bis R₈ = Wasserstoff und R₂ = Chlor] (Seite 2, Zeile 29 bis Seite 3, Zeile 4). Für die Wirkstoffe und das Polyethylenglykol sowie die weiteren Hilfsstoffe sind jedoch keine Mengenanteile angegeben, wobei auch nicht die Formulierungen angegeben sind, welche dieses bildet.

In DE 699 15 346 T2 sind Zusammensetzungen von Wirkstoffen in Kapseln beschrieben. Als Wirkstoffe sind unter einer sehr großen Zahl von aufgelisteten Wirkstoffen auch Clozapin und Olanzapin genannt, wobei angegeben ist, daß die direkte Verwendung einer flüssigen Form des Wirkstoffs möglich ist. Als Träger sind unter einer sehr großen Zahl von Trägern auch Polyethylenglykole mit einer Molmasse von 200, 300, 400 und höher und Propylenglykol sowie Ethanol genannt. Es fehlen jegliche Mengenangaben zu den Wirkstoffen und übrigen Bestandteilen.

In US 6,890,919 B2 ist eine Reihe von Wirkstoffen mit dem Clozapin ähnlicher Struktur von einer allgemeinen Formel umfaßt, wobei Clozapin selber nur als Vergleichssubstanz ohne Angabe einer Formulierung erscheint.

In WO 2005/063254 A2 sind Quetiapin und Olanzapin sowie ferner Clozapin ähnliche Verbindungen von einer allgemeinen Formel umfaßt, Clozapin ist aber ausgeschlossen. Es ist auch ein VPD Co-Lösungsmittel-System einer Lösung von 3 Gew.-% Benzylalkohol, 8 Gew.-% des nicht-polaren oberflächenaktiven Mittels Polysorbat 80 und 65 Gew.-% Polyethylenglykol 300, aufgefüllt mit absolutem Ethanol, was bedeutet, daß dessen Menge 24 Gew.-% beträgt, angegeben. Das ist aber noch nicht der ganze Träger, vielmehr ist zusätzlich noch eine wäßrige Phase angegeben.

In EP 0 958 822 A1 ist zwar eine Clozapintropfen-Lösung zur Verminderung des intraokulären Druckes beschrieben, als Lösungsmittel wird jedoch eine 0,9%-ige Natriumchloridlösung verwendet.

Aus US 2004/0120895 A1 sind bukkale Aerosolsprays bzw. Zungensprays, das heißt transmucosal anzuwendende Formulierungen mit einem Gehalt an einem Wirkstoff unter einer sehr großen Zahl von verschiedensten Wirkstoffen und darunter auch Clozapin bekannt. Diese können in einer Zusammensetzung aus Ethanol, Propylenglykol, Wasser und Aromastoffen sowie gegebenenfalls Polyethylenglykol vorliegen.

In EP 1 029 536 A1 sind gleiche Zusammensetzungen wie in US 2004/0120895 A1 beschrieben. Ferner ist angegeben, daß die Konzentrationen der Verunreinigungen, umfassend Oxidationsmittel, Reduktionsmittel, Lewis-Säuren oder -Basen und Wasser im Treibmittel weniger als 0,1% betragen sollen. Dieser Gehalt an Oxidationsmitteln ist jedoch, wie bereits gesagt, für die Treibmittel angegeben.

Demgegenüber liegt der Erfindung die Aufgabe zugrunde, die Verwendung von Zusammensetzungen von Clozapin, Quetiapin und seinen schwer oder sehr schwer löslichen Säureadditionssalzen und/oder Olanzapin zur Herstellung von Lösungen zur oralen Verabreichung, bei denen eine feinere Dosisabstimmung möglich ist und die eine optimale Stabilität aufweisen, zu schaffen.

Das wurde erfindungsgemäß überraschenderweise erreicht.

Gegenstand der Erfindung ist die Verwendung von Zusammensetzungen mit
a) 20 bis 100 mg/ml Clozapin, 25 bis 100 mg/ml Quetiapin, 25 bis 100 mg/ml (ausgedrückt als Quetiapinbase) von 1 oder mehr schwer oder sehr schwer löslichen Säureadditionssalz(en) von Quetiapin und/oder 1 bis 10 mg/ml Olanzapin,
b) 0 bis 20 mg/ml Benzylalkohol und/oder Phenylethylalkohol,
c) 25 bis 150 mg/ml Propylenglykol,
d) 0 bis 50 mg/ml von 1 oder mehr ätherischen Öl(en), Aroma bzw. Aromen und/oder Süßungsmittel (n) und
e) Rest bis je 1 ml von 1 oder mehr flüssigen Polyethylenglykol(en),
wobei an Stelle von c) oder zusätzlich zu c) bis zu 50 mg/ml Ethanol und/oder bis zu 0,5 Gew.-%, bezogen auf den/die Wirkstoff(e), von 1 oder mehr C₃₋₄-Alkanol(en) vorliegen können, zur Herstellung von Lösungen zur oralen Verabreichung. Das/die flüssige(n) Polyethylenglykol(e) liegt/liegen also in einem Mengenanteil, welcher der Rest zu 1 ml Lösung ist, vor.

Von WO 2005/007168 A1, in welcher es sich um Suspensionen handelt, unterscheidet sich die Erfindung grundlegend darin, daß erfindungsgemäß Lösungen vorgesehen sind, wobei diese überraschenderweise stabil sind. Daran ändert auch nichts die Erwähnung von Polyethylenglykol als Netzmittel, weil es sich dabei eben um ein Netzmittel und nicht um ein Lösungsmittel handelt.

Schon wegen der unspezifischen und damit nicht ausreichenden Offenbarung von Clozapin und Polyethylenglykol liegt WO 96/18622 von der Erfindung abseits.

Gegenüber der fehlenden Angabe der Mengenanteile von Clozapin und Polyethylenglykol in WO 96/18622 ist darauf hinzuweisen, daß es erfinderungsgemäß maßgeblich auch auf die Wahl der mengenmäßigen Zusammensetzung der zur Herstellung von Lösungen dienenden erfindungsgemäß verwendeten Zusammensetzungen ankommt, denn die korrekte Dosierbarkeit, die mittels Tropfen zu erfolgen hat, ist unterhalb und oberhalb des erfindungsgemäß festgelegten Bereiches, insbesondere des Bestandteiles e), vor allem des Polyethylenglykoles, beeinträchtigt und auch die Stabilität herabgesetzt. Es kann sich dabei eine völlige Unbrauchbarkeit ergeben. Auch bringt die Aufbewahrung in Kühlschränken die Unannehmlichkeit mit sich, daß die Zusammensetzung fest wird. Dabei ist vor allem der Mengenanteil des Bestandteiles e), insbesondere des Polyethylenglykoles, entscheidend; auch der Mengenanteil des Propylenglykoles spielt eine Rolle, wobei dieses aber bei bestimmten Zusammensetzungen ganz wegbleiben kann, wenn eine Kühlung der Lösung ausscheidet.

Schon wegen der unspezifischen und damit nicht ausreichenden Offenbarung von Clozapin und Olanzapin und Polyethylenglykolen in DE 699 15 346 T2 liegt diese von der Erfindung abseits.

Auch handelt es sich bei den Produkten der erfindungsgemäßen Verwendung im Gegensatz zu den Kapseln der letztgenannten Druckschrift um Tropfen. Abgesehen von der erfindungsgemäßen Wahl der herzustellenden Lösungszusammensetzung unterscheidet sich also die Erfindung von der genannten Druckschrift auch darin, daß die Wirkstoffe in Tropfen vorliegen. Bei den letzteren ist im Gegensatz zu den Kapseln bei der Wahl der Zusammensetzung auf die Dosiervorrichtung für eine exakte Dosierung Rücksicht zu nehmen. Tropflösungen müssen bestimmte physikalische Eigenschaften haben (die von der Zusammensetzung abhängig sind), die ein exaktes und komfortables Dosierungssystem (im vorliegenden Fall einen Tropfer) ermöglichen. Dagegen sind diese Eigenschaften für Lösungen in Kapseln ohne Bedeutung.

Für US 6,890,919 B2 gilt das für DE 699 15 346 T2 Gesagte analog.

Schon wegen der unspezifischen und damit nicht ausreichenden Offenbarung von Quetiapin und Olanzapin in WO 2005/063524 A2 liegt diese von der Erfindung, insoweit abseits. Von WO 2005/063524 A2 unterscheidet sich die Erfindung generell zunächst einmal darin, daß im Gegensatz zur ersteren in der durch die erfindungsgemäße Verwendung hergestellten Lösung kein oberflächenaktives Mittel Polysorbat 80 vorliegt. Das weist darauf hin, daß in WO 2005/063254 A2 der Wirkstoff in Polyethylenglykol nicht gelöst ist, sondern nur emulgiert. Beim "VPD Co-solvent system" in der genannten Druckschrift handelt es sich um einen Lösungsvermittler, der es gestatten soll, mit schlecht wasserlöslichen Substanzen eine wäßrige Lösung herzustellen. Dabei ist das Fehlen des Wassergehaltes gegenüber der letztgenannten Druckschrift ein weiterer wesentlicher Unterschied. Sofern erfindungsgemäß Propylenglykol vorliegt, ist das ein weiterer Unterschied zur letztgenannten Druckschrift.

Dadurch, daß in EP 0 958 822 A1 für Clozapin als Lösungsmittel eine 0,9%-ige Natriumchloridlösung und nur für Sulpirid Propylenglykol verwendet wurde, wurde der Fachmann davon abgelenkt, für Clozapin Propylenglykol oder ein anderes organisches Lösungsmittel zu verwenden.

Im Gegensatz zu US 2004/0120895 A1, welche transmucosal anzuwendende Formulierungen betrifft, ist die erfindungsgemäße Verwendung auf die Herstellung von Lösungen zur oralen Verabreichung abgestellt. Der Unterschied ist in der Fig. der letztgenannten Druckschrift anschaulich dargestellt. Im erstgenannten Fall erreicht der Wirkstoff durch die direkte Resorption an der Mucosa schnell (das heißt unter Umgehung des Schrittes der Dissolution im Magen und unter Umgehung des enterohepatischen Kreislaufs) und ohne Verluste durch den First-Pass-Metabolismus den systemischen Kreislauf, während im letztgenannten Fall eine solche Umgehung nicht stattfindet, vielmehr der Wirkstoff über den Magen in den Dünndarm, wo die Resorption in den enterohepatischen Kreislauf stattfindet, gelangt. Bei einer oral anzuwendenden Lösung, welche mit einer intramucosal zu verabreichenden nicht vergleichbar ist, ist daher die Wahl der Zusammensetzung kritischer.

Im Gegensatz zu den Clozapin-Formulierungen von US 2004/0120895 A1, welche zwingend Wasser enthalten, wurde durch die erfindungsgemäß verwendeten Zusammensetzungen dessen Vorliegen entbehrlich gemacht. Das bringt den Vorteil mit sich, daß der negative Einfluß des Wassers auf die Löslichkeit ausgeschaltet ist.

Für EP 1 029 536 A1 gilt das für US 2004/0120895 A1 Gesagte analog. Dabei ist in den Lösungen der erfindungsgemäßen Verwendung kein Treibmittel enthalten.

Vorzugsweise liegt das Clozapin in einem Mengenanteil von 25 bis 50 mg/ml, ganz besonders 40 bis 50 mg/ml, vor.

Zweckmäßig ist/sind das/die Säureadditionssalz(e) von Quetiapin Quetiapinfumarat und/oder Quetiapinmaleat.

Es ist auch bevorzugt, daß das/die Quetiapin und/oder Säureadditionssalz(e) von Quetiapin in einem Mengenanteil von 25 bis 50 mg/ml, jeweils ausgedrückt als Quetiapinbase, vorliegt.

Ferner ist es bevorzugt, daß das Olanzapin in einem Mengenanteil von 2,5 bis 5 mg/ml, vorliegt.

Für die allgemeinen Mengenanteilsbereiche sind bei Clozapin Dosierungsschritte von etwa 0,8 bis 4 mg, bei Quetiapin von etwa 1 bis 5 mg und bei Olanzapin von etwa 0,05 bis 0,5 mg, jeweils unter Verwendung einer Dosierungsvorrichtung, die 20 beziehungsweise 25 Tropfen pro ml Lösung erzielt, zweckmäßig. Beispielsweise bei einer bevorzugten Lösung von 25 mg/ml Clozapin sind Dosierungsschritte von 1 mg und bei einer ebenfalls bevorzugten Lösung von 50 mg/ml Clozapin 2 mg zweckmäßig. Bei besonders bevorzugten Lösungen von 50 mg/ml Quetiapin sind die zweckmäßigen Dosierungsschritte 2 mg. Bei besonders bevorzugten Lösungen von 2,5 beziehungsweise 5 mg/ml Olanzapin sind Dosierungsschritte von 0,1 beziehungsweise 0,2 mg zweckmäßig. Das Obige gilt jeweils für die Verwendung einer Dosierungsvorrichtung, die 20 beziehungsweie 25 Tropfen pro ml Lösung erzielt.

Vorzugsweise liegt/liegen der Benzylalkohol und/oder Phenylethylalkohol in einem Mengenanteil von 5 bis 15 mg/ml, insbesondere 10 mg/ml, vor.

Es ist auch bevorzugt, daß Propylenglykol in einem Mengenanteil von 50 bis 100 mg/ml, ganz besonders 75 mg/ml, vorliegt.

Ferner ist es bevorzugt, daß das/die ätherische(n) Öl(e) in einem Mengenanteil bis 25 mg/ml, insbesondere von 10 mg/ml, vorliegt/vorliegen.

Vorzugsweise ist das/ein ätherische(s) Öl Pfefferminzöl.

Ferner ist bevorzugt, daß das/die flüssige(n) Polyethylenglylcol (e) [ein] solche [s] mit einem Molekulargewicht von 200 bis 400, insbesondere 400 oder 300, ganz besonders 400, ist/sind.

Es ist auch bevorzugt, daß das gegebenenfalls enthaltene Ethanol in einem Mengenanteil bis zu 25 mg/ml, insbesondere bis zu 5 mg/ml vorliegt.

Die Erfindung wird an Hand der folgenden Beispiele näher erläutert.

**Beispiel 1**

| | |
|---|---|
| Clozapin | 25 mg |
| Benzylalkohol | 10 mg |
| Pfefferminzöl | 10 mg |
| Polyethylenglykol 400 | zum Auffüllen auf 1 ml |

Mit dieser Zusammensetzung und einem geeigneten Senkrechttropfer läßt sich eine Dosis von 1 mg Clozapin/Tropfen einstellen.

**Beispiel 2**

| | |
|---|---|
| Clozapin | 50 mg |
| Benzylalkohol | 10 mg |
| Polyethylenglykol 300 | zum Auffüllen auf 1 ml |

Mit dieser Zusammensetzung und einem geeigneten Senkrechttropfer läßt sich eine Dosis von 2mg/Tropfen einstellen.

**Beispiel 3**

| | |
|---|---|
| Clozapin | 40 mg |
| Benzylalkohol | 10 mg |
| Ethanol | 40 mg |
| Polyethylenglykol 400 | zum Auffüllen auf 1 ml |

Mit dieser Zusammensetzung und einem geeigneten Senkrechttropfer läßt sich eine Dosis von 2 mg/Tropfen einstellen.

**Beispiel 4**

| | |
|---|---|
| Clozapin | 25 mg |
| Benzylalkohol | 10 mg |
| Propylenglykol | 75 mg |
| Polyethylenglykol 400 | zum Auffüllen auf 1 ml |

Mit dieser Zusammensetzung und einem geeigneten Senkrechttropfer läßt sich eine Dosis von 1 mg/Tropfen einstellen.

**Beispiel 5**

| | |
|---|---|
| Clozapin | 50 mg |
| Benzylalkohol | 10 mg |
| Polyethylenglykol 400 | zum Auffüllen auf 1 ml |

Mit dieser Zusammensetzung und einem geeigneten Senkrechttropfer läßt sich eine Dosis von 1 mg Clozapin/Tropfen einstellen.

**Beispiel 6**

| | |
|---|---|
| Olanzapin | 2,5 mg |
| Benzylalkohol | 10 mg |
| Polyethylenglykol 300 | zum Auffüllen auf 1 ml |

Mit dieser Zusammensetzung und einem geeigneten Senkrechttropfer läßt sich eine Dosis von 0,1 mg Olanzapin/Tropfen einstellen.

**Beispiel 7**

| | |
|---|---|
| Quetiapin | 50 mg |
| Benzylalkohol | 10 mg |
| Ethanol | 25 mg |
| Pfefferminzöl | 10 mg |
| Polyethylenglykol 300 | zum Auffüllen auf 1 ml |

Mit dieser Zusammensetzung und einem geeigneten Senkrechttropfer läßt sich eine Dosis von 2 mg Quetiapin/Tropfen einstellen.

**Beispiel 8**

| | |
|---|---|
| Quetiapinfumarat (entsprechend 25 mg Quetiapin) | 28,8 mg |
| Phenylethylalkohol | 10 mg |
| Pfefferminzöl | 10 mg |
| Polyethylenglykol 400 | zum Auffüllen auf 1 ml |

## Patentansprüche

1. Verwendung von Zusammensetzungen mit
a) 20 bis 100 mg/ml Clozapin, 25 bis 100 mg/ml Quetiapin, 25 bis 100 mg/ml (ausgedrückt als Quetiapinbase) von 1 oder mehr schwer oder sehr schwer löslichen Säüreadditionssalz(en) von Quetiapin und/oder 1 bis 10 mg/ml Olanzapin,
b) 0 bis 20 mg/ml Benzylalkohol und/oder Phenylethylalkohol,
c) 25 bis 150 mg/ml Propylenglykol,
d) 0 bis 50 mg/ml von 1 oder mehr ätherischen Öl(en), Aroma bzw. Aromen und/oder Süßungsmittel(n) und
e) Rest bis je 1 ml von 1 oder mehr flüssigen Polyethylenglykol(en),
wobei an Stelle von c) oder zusätzlich zu c) bis zu 50 mg/ml Ethanol und/oder bis zu 0,5 Gew.-%, bezogen auf den/die Wirkstoff(e), von 1 oder mehr C₃₋₄-Alkanol(en) vorliegen können, zur Herstellung von Lösungen zur oralen Verabreichung.

2. Verwendung nach Anspruch 1, **dadurch gekennzeichnet, daß** das Clozapin in einem Mengenanteil von 25 bis 50 mg/ml vorliegt.

3. Verwendung nach Anspruch 1 oder 2, **dadurch gekennzeichnet, daß** das/die Säureadditionssalz(e) von Quetiapin Quetiapinfumarat und/oder Quetiapinmaleat ist/sind.

4. Verwendung nach Anspruch 1 bis 3, **dadurch gekennzeichnet, daß** das/die Quetiapin und/oder Säureadditionssalz(e) von Quetiapin in einem Mengenanteil von 25 bis 50 mg/M1, jeweils ausgedrückt als Quetiapinbase, vorliegt.

5. Verwendung nach Anspruch 1 bis 4, **dadurch gekennzeichnet, daß** das Olanzapin in einem Mengenanteil von 2,5 bis 5 mg/ml vorliegt.

6. Verwendung nach Anspruch 1 bis 5, **dadurch gekennzeichnet, daß** der Benzylalkohol und/oder Phenylethylalkohol in einem Mengenanteil von 5 bis 15 mg/ml, insbesondere 10 mg/ml, vorliegt/vorliegen.

7. Verwendung nach Anspruch 1 bis 6, **dadurch gekennzeichnet, daß** Propylenglykol in einem Mengenanteil von 50 bis 100 mg/ml vorliegt.

8. Verwendung nach Anspruch 1 bis 7, **dadurch gekennzeichnet, daß** das/die ätherische(n) Öl (e) in einem Mengenanteil bis 25 mg/ml, insbesondere von 10 mg/ml, vorliegt/vorliegen.

9. Verwendung nach Anspruch 1 bis 8, **dadurch gekennzeichnet, daß** das/ein ätherische[s] Öl Pfefferminzöl ist.

10. Verwendung nach Anspruch 1 bis 9, **dadurch gekennzeichnet, daß** das/die flüssige(n) Polyethylenglykol(e) [ein] solche[s] mit einem Molekulargewicht von 200 bis 400, insbesondere 400, ist/sind.
